# EUROPEAN PATENT SPECIFICATION

(11) **EP 2 541 248 B1**
(45) Date of publication and mention of the grant of the patent: **12.07.2017**
(21) Application number: 11747611.9
(22) Date of filing: 21.02.2011
(51) Int. Cl.: G01N 33/533, G01N 33/543

(54) **PREPARATION METHOD OF ANTIGEN-IMMOBILIZED IMMUNOFLUORESCENCE SLIDE, AND IMMUNOFLUORESCENCE SLIDE PREPARED THEREBY**
VERFAHREN ZUR HERSTELLUNG EINER ANTIGENIMMOBILISIERTEN IMMUNFLUORESZENZFOLIE UND IN DIESEM VERFAHREN HERGESTELLTE IMMUNFLUORESZENZFOLIE
PROCÉDÉ DE PRÉPARATION D'UNE LAME D'IMMUNOFLUORESCENCE À ANTIGÈNE IMMOBILISÉ ET LAME D'IMMUNOFLUORESCENCE PRÉPARÉE SELON CE PROCÉDÉ

(30) Priority: 23.02.2010 KR 20100016157
(43) Date of publication of application: 02.01.2013
(73) Proprietor: Korea Food Research Institute, Gyeonggi-do 463-746 (KR)
(72) Inventor: KIM, Nam Soo, Seoul 136-110 (KR); CHO, Young Jin, Seoul 138-220 (KR); KIM, Chong Tai, Yongin-si Gyeonggi-do 302-205 (KR)
(74) Representative: Engelhard, Markus
(86) International application number: PCT/KR2011/001126
(87) International publication number: WO 2011/105721

(56) References cited:
- KR-A- 20090 073 669
- KR-B1- 100 620 959
- US-A1- 2007 048 807
- HAI LI ET AL: "Aminosilane Micropatterns on Hydroxyl-Terminated Substrates: Fabrication and Applications", LANGMUIR, vol. 26, no. 8, 30 November 2009 (2009-11-30), pages 5603-5609, XP055025622, ISSN: 0743-7463, DOI: 10.1021/la9039144
- KIM N S ET AL: "Glass Slide-based Immunosensing for C-Reactive Protein Using Quantum Dot-Antibody Conjugate", SAN'EOB SIGPUM GGONGHAG = FOOD ENGINEERING PROGRESS, HAN'GUG SAN'EOB SIGPUM GONGHAGHOE, REPUBLIC OF KOREA, vol. 14, no. 1, February 2010 (2010-02), pages 21-26, XP009170719, ISSN: 1226-4768
- LEE, YOONSUK ET AL.: 'Proteochip: A highly sensitive protein microarray prepared by a novel method of protein immobilization for application of protein-protein interaction studies' PROTEOMICS vol. 3, March 2003, pages 2289 - 2304, XP008057185
- JUNG Y ET AL.: 'Controlled antibody immobilization onto immunoanalytical platforms by synthetic peptide' ANAL BIOCHEM. vol. 374, no. 1, 01 March 2008, pages 99 - 105, XP022437940
- STADTHERR K ET AL: "AN APTAMER-BASED PROTEIN BIOCHIP", ANALYTICAL CHEMISTRY, AMERICAN CHEMICAL SOCIETY, vol. 77, no. 11, 1 June 2005 (2005-06-01), pages 3437-3443, XP002394235, ISSN: 0003-2700, DOI: 10.1021/AC0483421

## Description

### TECHNICAL FIELD

The present invention relates to a method of preparing an antigen-immobilized immuno-fluorescence slide including: immobilizing a C-reactive protein on a slide to prepare a protein chip, mixing an antibody that specifically binds to a target protein, with streptavidin, to label the antibody with a fluorescent nanoparticle, immune-reacting the antibody by competitive mixing, assaying with a fluorescence camera, and an immuno-fluorescence slide made by using the method.

### BACKGROUND ART

Biochips include various kinds of probes immobilized on a unit area of a surface of a solid support, and when such biochips are used, only with a small amount of sample, disease diagnosis, high-throughput screening (HTS), and enzyme activity measuring may be easily performed in a large scale.

Most of methods of immobilizing a probe on a slide include immobilizing a probe on a glass slide pre-treated with a coating material. For these methods, various surface chemical materials have been proposed, and for example, a self-assembled monolayer may be used (Korean Patent Publication No. 2003-0038932).

In this regard, as a trial to increase an amount of an immobilized probe and to maintain the activity of a probe, a 3-dimensional (3D) immobilization method was developed (Gill and Ballesteros, Trends in Biotechnology 18:282, 2000). Examples of such a 3D immobilization method are a method using a Hydrogel^{®} coating slide of Packard Bioscience, a method using a polyethyleneglycol-based hydrogel of Biocept Company, and a method using solgel of LG Chemical Co., Ltd, and the like.

A slide for use in an immuno-sensor is selected from a group consisting of glass, silicon, hydrogel, metal, ceramic, and a porous membrane.

Currently, an antibody or antigen probe-plate made by immobilizing an antibody or an antigen on a substrate, which is based on a specific reactivity of an antigen or an antibody, is widely used for detection. Representative kits for such use are a rapid test kit, a routine test kit, and a biochip kit.

These kits are used to detect a target antigen or target antibody corresponding to an immobilized antibody probe or antigen probe.

In some cases, various reaction groups are assembled onto a single probe-plate, and each of those reaction groups includes a test strip for the detection of an antigen or antibody.

Meanwhile, as the Health Functional Food Act has taken into effect, even in Korea, it is possible to manufacture natural material-based food materials and processed food and to obtain recognition thereof as health functional food. However, to do this, materials for technically proving the functionality of health functional food, for example, cardiovascular disease prevention, aging suppression, cancer prevention, obesity prevention, immune controlling, or gastro-intestinal tracts related disease prevention, need to be presented. Accordingly, food-function evaluating is being considered more important, and as a result, evaluating of functions has a high likelihood of commercialization.

Food-function evaluating includes *in vitro* function evaluation, *in vivo* function evaluation using a lab animal, such as a rat, and clinical trials, performed based on those six function types, and in terms of the food industry, a demand for developing an *in vivo* function detection techniques using a lab animal for effectively and rapidly performing function evaluation of food materials and functional good increases.

To be recognized as a functional food, a target function, such as a cardiovascular function, needs to be scientifically proved, and it cannot be stressed too much the importance of food-function evaluation (Kim et al., Detecting C-reactive protein by using direct binding quartz crystal microbalance immunosensor, Journal of The Korean Society for Biotechnology and Bioengineering, Vol. 22, p. 443, 2007). Conventionally, *in-vivo* function evaluation using lab animals, which is performed prior to clinical trials, is to evaluate change in bodily elements, such as body weight, blood pressure, and shape of organs. However, this method causes non-uniform results, and also, requires variability of evaluation protocols, skilled experts, and high-cost assay instruments. A uniform *in vivo* food-function evaluation method is to measure an increase or decrease of a particular biomarker protein related to a particular disease or metabolism symptoms in a lab animal, such as a rat. In this regard, what contains a food that is desired to obtain recognition as a functional food is administered to the lab animal.

Korea Registered Patent 10-921237 discloses, as an *in vivo* function evaluation method for rapidly detecting a cardiovascular function of a food material and a functional food, a method of detecting with high sensitivity a C-reactive protein (Biosensors and Bioelectronics, Vol. 19, p. 1193, 2004; Clinical Chemistry, Vol. 47, p. 403, 2001; New England Journal of Medicine, Vol. 340, p. 448, 1999; Biochemical Journal, Vol. 327, p. 425, 1997) by using a quartz crystal microbalance immunosensor, wherein the C-reactive protein is a pentameric protein that is known as a major biomarker of coronary artery disease, hypertension, or the like, that is synthesized by induction-stimulation by interleukin-6 and interleukin-1β in the liver of a mammal, and that has a molecular mass of about 118 killodalton (kDa).

This method, compared to an enzyme-linked immunosorbent assay (ELISA) (American Journal of C ardiology, Vol. 1, p. 155, 2005; American Journal of Veterinary Research, Vol. 1, p. 62, 2005; Journal of Clinical Laboratory Analysis, Vol. 18, p. 280, 2004) for evaluating a C-reactive protein, is easily used without measurement disturbances by a coloring material, and also, has better or equal measurement sensitivity than typical other sensor measurement methods (Biosensors and Bioelectronics, Vol. 22, p. 973, 2007; Biosensors and Bioelectronics, Vol. 21, p. 1987, 2006; Biosensors and Bioelectronics, Vol. 21, p. 1631, 2006; Biosensors and Bioelectronics, Vol. 21, p. 1141, 2006; Analytical Biochemistry, Vol. 328, p. 210, 2004).

Recently, sensor measurement using a biosensor more frequently uses a nanomaterial, such as metallic colloid, carbon nanotube, fluorescent silica nanoparticle, or semiconductor quantum dot, and the use of such nanomaterials contributes to an increase in sensitivity, stability, or selectivity of sensor signals (Analytical Chemistry, Vol. 79, p. 630-707, 2007; Biosensors and Bioelectronics, Vol. 21, p. 1900, 2006; Langmuir, Vol. 22, p. 4357, 2006; Nano Letters, Vol. 5, p. 113, 2005; Biosensors and Bioelectronics, Vol. 20, p. 2454, 2005; Proceedings of the National Academy of Sciences, Vol. 100, p. 4984, 2003; Biochemical and Biophysical Research Communications, Vol. 274, p. 817, 2000).

When an *in vivo* cardiovascular functionality of a food is evaluated by measuring a C-reactive protein as a biomarker present in blood of a lab animal by using a biosensor, an increase in sensor sensitivity is still a critical issue and also, ease of measurement is endlessly pursued.

KR 2009 0073669 A describes a crystal oscillator immune sensor.

HAI LI ET AL: LANGMUIR, vol. 26, no. 8, pages 5603-5609, relates to aminosilane micropatterns on hydroxyl-terminated substrates.

KIM N S ET AL: FOOD ENGINEERING PROGRESS, vol. 14, no. 1, pages 21-26, relates to glass slide-based immunosensing.

LEE, YOONSUK ET AL: PROTEOMICS, vol. 3, pages 2289-2304, describes a protein microarray.

JUNG Y ET AL.: ANAL BIOCHEM., vol. 374, no. 1, pages 99-105, describes a strategy to immobilize an antibody on various sensor surfaces.

STADTHERR K ET AL.: ANALYTICAL CHEMISTRY, vol. 77, no. 11, pages 3437-3443, relates to an aptamer-based protein biochip.

### DETAILED DESCRIPTION OF THE INVENTION

### TECHNICAL PROBLEM

The present invention provides a dispensable immunosensor slide with high sensor sensitivity, which allows a user, without a complicated apparatus, such as a biochip arrayer or a micro-array scanner, to immobilize a certain volume of an antigen on a slide with a micro dispensing pipette in manufacturing or measuring a biochip to perform an immunoreaction between a target protein and an zygocyte of an antibody and a fluorescent nanoparticle, or an immunoreaction between an immobilized antigen and a mixture including a fluorescent nano-probe and a particular target molecule on an antigen coating site of the immuno-fluorescence slide, and then, fluorescent intensity or the number of fluorescence particles of the nano-probe that specifically binds to the surface of the slide by the immunoreaction is measured using a fluorescence microscope so as to identify the concentration of a target molecule, such as a body indicator.

### TECHNICAL SOLUTION

The present invention relates to a method in accordance with claims 1, 3 and 4 and to a C-reactive-protein-immobilized immune-fluorescence slide in accordance with claim 2.

According to the present invention, a target protein (antigen) is immobilized on a slide, an antibody that specifically binds to the antigen is labeled with a fluorescent material, a target protein collected from a specimen is mixed with the fluorescence-labeled antibody, the mixture is reacted with the immobilized antigen site on the slide, unreacted antibody and antigen are washed with distilled water, the slide is placed in an incubator for drying, and fluorescent intensity or the number of fluorescence particles is measured by using a fluorescence microscope.

To assay a sample, according to the present invention, an indirect-competitive assay format is used. However, one of ordinary skill in the art may easily know that the present invention may also be embodied by using a direct-competitive assay format or a sandwich assay format.

An immunosensor slide used in embodiments of the present invention may be selected from the group consisting of glass, silicon, hydrogel, metal, ceramic, and a porous membrane, and may have a size of 20 to 40 X 70 to 80 mm.

In the present invention, a C-reactive protein (hereinafter referred to as "CRP") is used as a target sample.

A C-reactive protein antibody may be any one of antibodies which are prepared by using C-reactive proteins induced from a mammal, such as a rat, a mouse, a rabbit, a monkey, or a human being.

A sample collected from a specimen may be any one of a reference solution in which a C-reactive protein is dissolved in a reaction buffer solution, and a tissue extract collected from blood, serum, blood plasma, saliva, body fluid, and the liver.

As a solution for blocking a portion of the surface of the slide which does not react with an antigen, any one of a 1-5% bovine serum albumin (BSA) solution, a 1-5% rat serum albumin (RSA) solution, a 1-5% human serum albumin (HSA) solution, a 1-5% mouse serum albumin (MSA) solution, and a 1-5% goat serum albumin (GSA) solution.

Hereinafter, each step of the method according to the present invention is described in detail.

Immobilizing of a C-reactive protein as an antigen on a slide may be performed by the following method.

As for the immobilizing method, the immobilizing of an antigen may be performed by using 3-aminopropyltrimethoxysilane (APTMS). For this method, first, a modified slide is prepared using APTMS. For the modification, a slide is immersed in a piranha solution (H₂SO₄:H₂O₂ = 2:1 to 4:1) for 5 to 15 minutes, and then, the slide is washed with distilled water, is sonicated in distilled water for 3 to 10 minutes, is washed by hydration with hot water having a temperature of 85 to 95 °C for 30 to 90 minutes, and then, is dried at room temperature on kimwipes for 20 to 120 minutes. The surface-washed slide was placed in a petri dish containing a solution in which 5 to 15% of APTMS is dissolved in acetone, and then, a reaction is performed at room temperature for 30 to 90 minutes. Front and rear sides of the slide are sequentially washed three to four times with acetone, a 30-70 mM phosphate buffer solution (pH 6.5-7.8), and a distilled water, and then, the slide is immersed in a beaker containing distilled water for 1 to 60 minutes. The slide is dried on kimwipes at room temperature for 20 to 120 minutes, heat treated at a temperature of 30 to 150°C in a convection oven for 3 to 7 hours, is left to sit for cooling, and then preserved in a dried state in a desiccator until use.

The slide modified with APTMS is hydrated by immersion in distilled water for 10 to 20 minutes. The immersed slide is placed in a petri dish containing a solution including 1 to 4% glutaraldehyde and a reaction is performed for 30 to 90 minutes to activate the slide so that a protein is bindable to the surface of the slide. Front and rear surfaces of the activated slide are washed three to four times by using a 30-70 mM phosphate buffer solution (pH 6.5-7.8). Then, the slide is immersed in a beaker containing distilled water for 1 to 60 minutes. The slide is taken away from the beaker and placed on kimwipes and dried for 20 to 120 minutes at room temperature.

The glutaraldehyde used above is prepared by adding 1 to 4 mℓ of 30-70% glutaraldehyde to 46 to 49 mℓ of distilled water, and 30 to 70 mℓ of the prepared solution is used.

To immobilize a target protein, for example, a C-reactive protein, first, 0.01 to 0.5 mg/mℓ of a C-reactive protein is dissolved in a 30-70 mM phosphate buffer solution (pH 6.5-7.8) to prepare an antigen solution for immobilization. A petri dish containing the glutaraldehyde-activated slide is placed on a spotting guide having a lattice shape, and spotting is performed with 1 to 100 *µ*ℓ of the previously prepared antigen solution on a portion of the slide corresponding to a spot point on the spotting guide, and then, the resultant structure is covered with a cover and a reaction is performed for 1 to 6 hours to immobilize the antigen. Then, front and rear surfaces of the slide are washed three to four times with 30-70 mM phosphate buffer solution (pH 6.5-7.8), and then, immersed in a beaker containing distilled water for 1 minute. To block a portion of the surface of the glutaraldehyde-activated slide subjected to the immobilizing of the antigen which does not react with the antigen on the surface, the slide is placed in a small plastic petri dish containing 1 to 5% BSA solution dissolved in a 30-70 mM phosphate buffer solution (pH 6.5-7.8), and then, the resultant structure is covered with a cover and a reaction is performed for 1 to 5 hours, and when the reaction is stopped, front and rear surfaces of the slide blocked with BSA are washed three to four times with 30-70 mM phosphate buffer solution (pH 6.5-7.8), and then, the slide is immersed in a beaker containing distilled water for 1-60 minutes. The slide is placed on kimwipes and dried for 20-120 minutes at room temperature.

Also disclosed is a second immobilizing method which does not form part of the present invention, in which the immobilizing of an antigen may be performed by using 3-mercaptopropyltrimethoxysilane-N-gamma- maleimidobutyryloxy succinimide ester (MPTMS-GMBS). First, for surface cleaning, a slide is immersed in a mixed solution (HCl:MtOH = 1:1 to 1:2, a volumetric ratio) including a strong hydrochloric acid (18-36%) and methanol (50-100%) for 15 to 45 minutes, and then, front and rear surfaces of the slide are washed three to four times with distilled water. After the washing of the slide with 85 to 95 °C of distilled water, the slide is placed on kimwipes and dried for 20 to 120 minutes at room temperature.

To silanize the resultant slide, 50 to 500 mℓ of an MPTMS solution in which 1-3% MPTMS is dissolved in an organic solvent solution selected from the group consisting of toluene, dimethyl foramide, and acetone is prepared. The slide is placed in a petri dish containing the solution and a reaction is performed at room temperature for 1 to 3 hours. Front and rear surfaces of the slide are washed three to four times with the same organic solvent as used in preparing the MPTMS solution, and the slide is placed on kimwipes and dried for 20 to 120 minutes at room temperature.

To activate the surface of the slide, an 1 to 3 mM GMBS solution is prepared as described below. 14 to 42 mg of a GMBS sample is dissolved in 100 *µℓ* of N,N-dimethylformamide (DMF; Sigma-Aldrich Company, USA), and then, 49.9 mℓ of ethanol is added thereto. The slide subjected to the silanization is placed in a petri dish containing the GMBS solution and a reaction is performed at room temperature for 30 to 90 minutes. Front and rear surfaces of the slide are washed three to four times with distilled water and a 30-70 mM phosphate buffer solution (pH 6.5-7.8). Then, the slide is placed on kimwipes and dried for 20 to 120 minutes at room temperature.

A CRP antigen is immobilized in the following manner. First, a C-reactive protein is dissolved at a concentration of 0.01 to 0.5 mg/mℓ in a 30-70 mM phosphate buffer solution (pH 6.5-7.8) to prepare an antigen solution for immobilization. A petri dish containing a GMBS-activated slide is placed on a spotting guide, and then, 1 to 100*µ*ℓ of the previously prepared antigen solution is spotted on a portion of the slide corresponding to a spot point of the spotting guide and then, the resultant structure is covered with a cover and a reaction is performed for 1 to 6 hours to immobilize the antigen. Then, front and rear sides of the slide are washed three to four times with a 30-70 mM phosphate buffer solution (pH 6.5-7.8), and the slide is immersed in a beaker containing distilled water for 1 to 60 minutes. To increase assay accuracy, the slide is placed on a small plastic petri dish containing a 1 to 5% BSA solution and the resultant structure is covered with a cover and a reaction is performed for 1 to 5 hours. Front and rear surfaces of the slide are washed three to four times with the phosphate buffer solution described above. Then, the slide is immersed in a beaker containing distilled water for 1 to 60 minutes, and then, placed on kimwipes and dried for 20 to 120 minutes at room temperature.

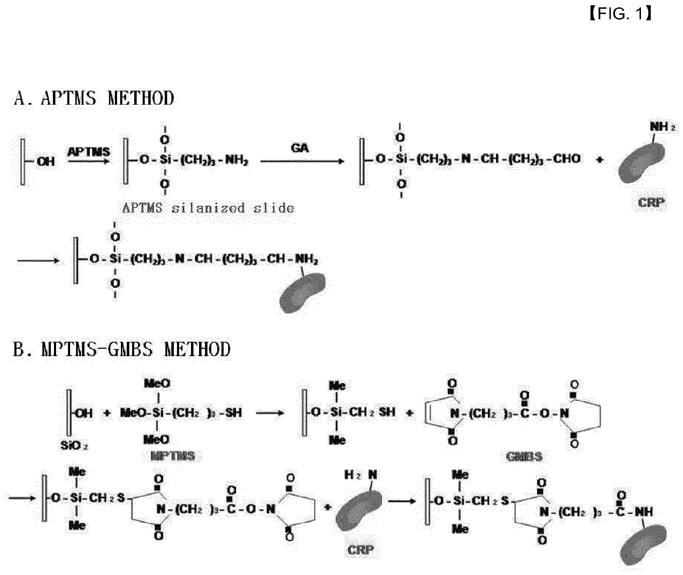

These two methods are schematically illustrated in FIG. 1

As for assaying a particular protein by using an immunosensor slide according to the present invention, an antibody that specifically binds to an antigen immobilized on the slide is labeled. The labeling of an antibody may be performed by using a fluorescent staining method with high sensitivity and reproducibility, and to do this, according to the present invention, a fluorescent silica nanoparticle (hereinafter referred to as "FSNP") is used.

A method of preparing FSNP is briefly described below. FSNP, which is used to label an antibody that specifically binds to an antigen, may be prepared by using a dye entrapment method in which an organic fluorescence pigment is included in a silica structure, a core shell method in which an active organic fluorescence pigment is covalently bonded to an amino silane compound and the result structure is included in a silica structure, or an inverted core shell method in which a organic fluorescence pigment is included in a silica structure and the formed particle is reacted with an organosilane compound to introduce a functional group to obtain functionality. In this regard, examples of such an organic fluorescence pigment are dichlorotris(1,10-phenanthroline)ruthenium(II)·hydrate, fluorescein, rhodamine B, and 5(6)-Carboxytetramethylrhodamine, and examples of an active organic fluorescence pigment are fluorescein isothiocyanate, 5(6)-carboxytetramethylrhodamine N-succinimidyl ester, tetramethylrhodamine 5-isothiocyanate, bis(2,2'-bipyridine)-4'-methyl-4-carboxybipyridine-ruthenium N-succinimidyl ester-bis(hexafluorophosphate), and bis(2,2'-bipyridine)-4,4'-dicarboxybipyridine ruthenium di(N-Succinimidyl ester)bis(hexafluorophosphate), an example of an amino silane compound is (3-aminopropyl)triethoxysilane, and examples of an organosilane compound are compounds including carboxylate, amine, amine/phosphonate, poly(ethylene glycol), and octadecyl, carboxylate/ octadecyl functional groups.

A method of labeling an antibody with FSNP is described below. First, FSNP needs to be modified with streptavidin (SA) which specifically binds to biotin. This modification is performed to bind SA-bonded FSNP, which is to be described later, to a biotinylated antibody. To modified FSNP with SA, first, FSNP is reacted with (3-mercaptopropyl)trimethoxy silane (MPTMS) to prepare a thiol-modified FSNP. 1 to 5 mg of FSNP and 5 to 15 mℓ of an alcohol, such as ethanol, methanol, or propanol, are added to a cap tube having a capacity of 10 to 70 mℓ and the mixture is shaken well and then, sonicated to completely dissolve FSNP in the alcohol for 10 to 30 minutes. Thereafter, 30-70 to 200 *µℓ* of MPTMS is added to the cap tube, and while the resultant mixture is stirred at a temperature of room temperature at a low speed of 30-200 rpm, a reaction is performed for 2 to 6 hours. The reaction mixture is centrifuged at a temperature of 3 to 10 °C for 20 to 40 minutes at a speed of 7000-17000 rpm and the obtained precipitate is washed one to five times by using the same alcohol as used for the reaction, and following each washing, centrifuging is performed under the same conditions as described above to obtain a precipitate. A cap is separated from the tube and the tube is softly covered with an aluminum foil and dried at room temperature. The precipitate is dissolved in 2 to 6 mℓ of a 30-70 mM phosphate buffer solution (pH 6.5-7.8) to prepare a thiol-modified FSNP solution.

A maleimide-activated SA is added to the thiol-modified FSNP to prepare SA-bonded FSNP. In detail, 0.1 to 0.5 mg of SA-maleimide is dissolved in 3 to 7 mℓ of a 30-70 mM phosphate buffer solution (pH 6.5-7.8) in a cap tube having a capacity of 10-70 mℓ to prepare a SA-maleimide solution. Then, 0.5 to 2.0 mℓ of the thiol-modified FSNP solution is added thereto, and while the mixture is continuously stirred at a low speed of 30 to 200 rpm, a reaction is performed at room temperature for 1 to 3 hours. The reaction product is centrifuged at a temperature of 3 to 10 °C for 20 to 40 minutes at a speed of 7000-17000 rpm to obtain a precipitate. The precipitate is washed one to five times with 30 to 70 mM phosphate buffer solution (pH 6.5-7.8), and following each washing, centrifuging is performed under the same conditions as described above to obtain a precipitate. The precipitate is re-suspended with 1 to 5 mℓ of 30-70 mM phosphate buffer solution (pH 6.5-7.8). The suspension is loaded into a cap tube, wrapped with an aluminum foil, refrigerated, and reacted with a biotinylated antibody, which is described later.

Biotinylating a C-reactive protein antibody may be performed by using one of the following methods.

A method of biotinylating a C-reactive protein antibody includes: adding a carbonate-bicarbonate buffer to a C-reactive protein antibody to obtain a C-reactive protein antibody-containing carbonate-bicarbonate buffer, reacting a biotinamidohexanoic acid N-hydroxysuccinimide ester (NHS-LC-biotin) solution, which is a biotinylation reagent dissolved in dimethyl formamide that is an organic solvent, with the carbonate-bicarbonate buffer, and dialyzing the obtained solution in a sodium chloride solution to remove an unreacted biotinylation reagent.

In detail, the biotinylating method includes adding a carbonate-bicarbonate buffer to a C-reactive protein antibody to obtain a carbonate-bicarbonate buffer containing 1.0 to 3.0 mg/mℓ C-reactive protein antibody, reacting 30 to 100 *µ*ℓ of NHS-LC-biotin solution in which 1 to 5 mg/mℓ NHS-LC-biotin is dissolved in dimethyl formamid with 25 to 75 *µ*ℓ of a carbonate-bicarbonate buffer for 1 to 5 hours in iced water, and dialyzing 75 to 175 *µ*ℓ of the obtained solution in 0.1 to 0.3M sodium chloride solution overnight to remove an unreacted biotinylation reagent.

A method of biotinylating a C-reactive protein antibody includes: adding a phosphate buffered saline to a C-reactive protein antibody to prepare a C-reactive protein antibody-containing phosphate buffered saline, reacting a sulfosuccinimidyl-6-(biotinamido)hexanoate (sulfo-NHS-LC-biotin) solution, which is a water-soluble biotinylation reagent dissolved in redistilled water, with the phosphate buffered saline, and dialyzing the obtained solution in a phosphate buffered saline to remove an unreacted biotinylation reagent.

In detail, the biotinylating method according to the present embodiment includes adding a phosphate buffered saline to a C-reactive protein antibody to prepare a phosphate buffered saline solution in which the concentration of the C-reactive protein antibody is in a range of 0.5 to 3.0 mg/mℓ, reacting 5 to 20 *µ*ℓ of a sulfo-NHS-LC-biotin solution in which 2 to 20 mM sulfo-NHS-LC-biotin is dissolved in redistilled water with 30 to 800 *µ*ℓ of the phosphate buffered saline in iced water for 1 to 5 hours, and dialyzing 55 to 820 *µ*ℓ of the resultant solution in 0.05 to 0.25M phosphate buffered saline overnight to remove an unreacted biotinylation reagent.

Next, an antibody to which a nanomaterial is bonded is prepared. When the SA-modified FSNP is mixed with the biotinylated antibody, due to specific bonding between biotin and SA, a fluorescent material-labeled antibody is prepared.

In detail, the SA-modified FSNP solution is sonicated for 10 to 30 minutes, and then, 200 to 600 *µ*ℓ of the obtained solution is added to 1 to 3 mℓ of 0.05 to 0.25 mg/mℓ biotinylated antibody solution. While the mixture is stirred at time intervals of 30 minutes at a low speed of 30 to 200 rpm for 2 to 7 minutes, a reaction is performed at room temperature for 1 to 3 hours. The reaction product is centrifuged at a temperature of 3 to 10°C for 20 to 40 minutes at a rate of 7000-17000 rpm, and a supernatant is discarded and a precipitate is used. The precipitate is washed one to five times with 30 to 70 mM phosphate buffer solution (pH 6.5-7.8), and following each washing, centrifuging is performed under the same conditions as described above and the obtained precipitate is used. The precipitate is re-suspended in 0.4 to 1.2 mℓ of 30-70 mM phosphate buffer solution (pH 6.5-7.8).

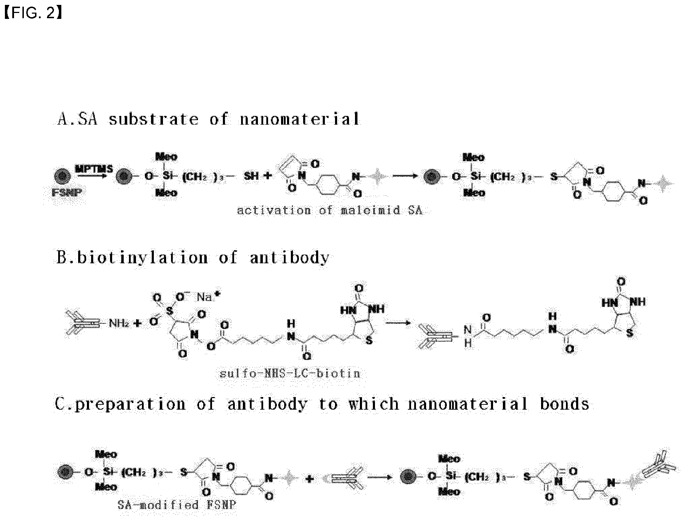

A schematic method of labeling an antibody that specifically binds to CRP with a fluorescent material, according to the present invention, is illustrated in FIG. 2.

In assaying a particular protein by using an immunosensor slide according to the present invention, a target protein obtained from a specimen is mixed with the labeled antibody.

In detail, a C-reactive protein sample solution is prepared in various concentrations by using a 30-70 mM phosphate buffer solution (pH 6.5-7.8) in an eppendorf tube, and a C-reactive protein antibody solution which is prepared by specific binding between biotin and SA and to which FSNP is bonded as a nanomaterial, is added to the various concentrations of the C-reactive protein sample solution, wherein amounts of the C-reactive protein antibody solution and the C-reactive protein sample solution are the same, and the mixture remains stationary at room temperature for 5 to 300 minutes.

Next, the mixed mixture is reacted with an antigen immobilized on the slide.

In detail, a petri dish containing a slide as a bio-transducer on which a C-reactive protein as an antigen is immobilized by the glutaraldehyde process or the MPTMS-GMBS process and adsorption of a non-selective protein is minimized by the bovine serum albumin (BSA) blocking process, is placed on a spotting guide having a lattice structure, and the mixing is performed where the antigen is immobilized on the slide, and then, 1 to 100 *µ*ℓ of a mixture including a target protein obtained from a specimen which has been stationary at room temperature for 5 to 300 minutes and the labeled antibody is accurately spotted, and the resultant structure is covered with a cover, and a reaction is performed at room temperature for 0.5 to 16 hours, thereby performing an indirect-competitive assay between the antigen immobilized on the slide and a target protein collected from a specimen against the FSNP-labeled antibody. After a reaction is stopped, unreacted antibody and antigen are removed therefrom by washing front and rear sides of the slide three to four times with distilled water and immersing the slide in a beaker containing distilled water for 1 to 60 minutes.

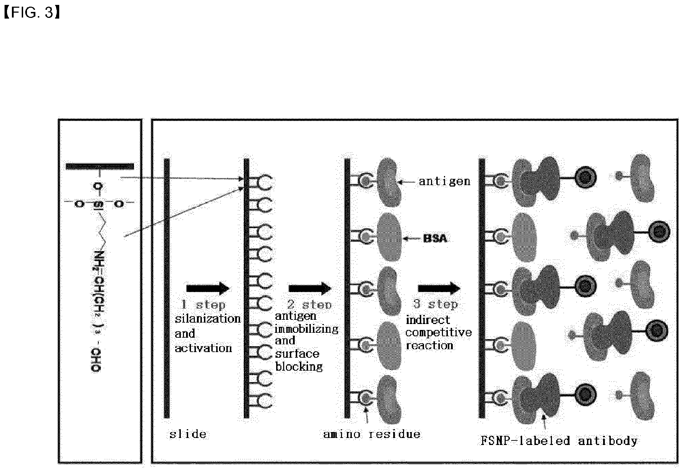

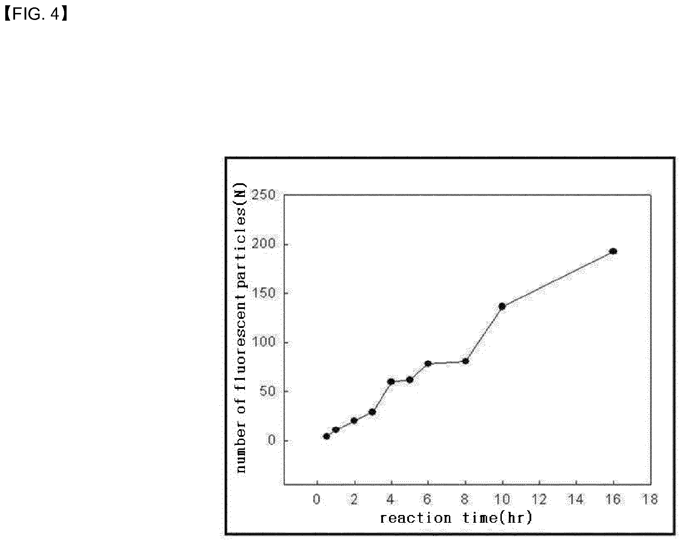

FIG. 3 illustrates a diagram for explaining how the antigen immobilized on a slide binds to the fluorescence-labeled antibody by indirect-competitive assay, and FIG. 4 shows a graph of a change of the number of fluorescent particles binding to a slide when instead of a C-reactive protein collected from a specimen, a 30-70 mM phosphate buffer solution (pH 6.5-7.8) is mixed with the labeled antibody as described above, and then, the mixture is spotted on the antigen immobilized on the slide to perform an antigen-antibody reaction over time.

The slide is dried in an incubator for 20 to 120 minutes.

Fluorescence intensity and the number of fluorescent particles are measured by using a fluorescence microscope. When the concentration of CRP in a specimen is high, more CRP reacts with fluorescence-labeled antibodies. Accordingly, less fluorescence-labeled antibodies bind to the antigen immobilized on the slide, and thus, fluorescence intensity and the number of fluorescent particles are reduced. As described above, an antigen immobilized on a slide and an antigen obtained from a specimen competitively interacts with a fluorescence-labeled antibody, thereby showing different intensity levels of fluorescence. Thus, CRP can be easily measured for a relatively short period of time.

### ADVANTAGEOUS EFFECTS

The present invention provides an immunosensor slide for easily and highly-sensitively measuring a biomarker which is an *in vivo* food-function indicator by a nanomole-level of evaluation by coating a C-reactive protein, which is known as a major biomarker for coronary artery disease, hypertension, and inflammation, on the slide and reacting the C-reactive protein with an antibody binding to a fluorescent nanoprobe.

The present invention may establish a future food-function evaluation related basic technique that is required for a supersensitive, high-speed simultaneous measurement on, in addition to a C-reactive protein, a biomarker, such as low density lipoprotein (LDL), fibrinogen, or angiotensin II, existing in a wide concentration range in, for example, blood of a lab animal.

### DESCRIPTION OF THE DRAWINGS

FIG. 1 is a schematic view illustrating two methods of immobilizing a C-reactive protein on a slide.
FIG. 2 is a schematic view illustrating a method of labeling an antibody that specifically binds to CRP with a fluorescent material.
FIG. 3 is a schematic view illustrating binding of a fluorescence-labeled antibody to an antigen immobilized on the slide by indirect competition.
FIG. 4 shows a graph of a change of the number of fluorescent particles binding to a slide when instead of a C-reactive protein collected from a specimen, a 30-70 mM phosphate buffer solution (pH 6.5-7.8) is mixed with the labeled antibody as described above, and then, the mixture is spotted where the antigen is immobilized on the slide to perform an antigen-antibody reaction over time.

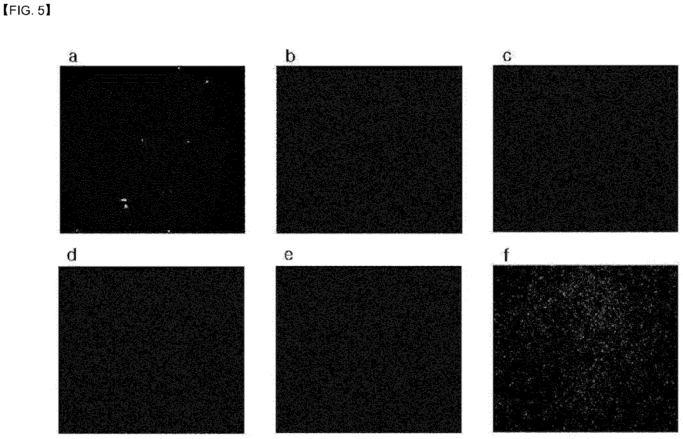

FIGS. 5A, 5B, and 5C show images showing that inhibitory spots of a contaminating material, such as impurities, present on the surface of a slide before treatment with a piranha solution are removed by a piranha treatment, and even after silanization with APTMS, inhibitory spots are not observed. FIG. 5D shows an image of a substrate treated with a 2.5% glutaraldehyde solution in which inhibitory spots are not observed. FIG. 5E shows an image in which inhibitory spots are not present on a BSA-blocked portion of the surface of the slide after an antigen is immobilized. FIG. 5F shows an image containing fluorescent spots of a fluorescent silica nanoparticle-labeled antibody binding to the surface of the slide subjected to an indirect competitive reaction.
FIG. 6 is a view illustrating an apparatus for fluorescent assay.
FIG. 7 illustrates a graph of a calibration curve of an antigen-immobilized immuno-fluorescence slide and a FSNP-labeled antibody-based C-reactive protein.

### MODE OF THE INVENTION

Hereinafter, the method according to the present invention is described in detail with reference to examples. However, the scope of the present invention is not limited to the examples.

Materials used in the present experiments are described as follows:
A recombined histidine tagged rate CRP (pure state) expressed in a mouse myeloma cell line (NSO) was obtained from R&D Systems, Inc. (Miniapolice, MN, USA) for use throughout the present experiments. A homopentameric structure thereof consisted of three non-covalent and two covalent sub units. In addition, a monoclonal anti-rat CRP antibody generated from a hybridoma obtained from a fusion of mouse myeloma and a B cell that is obtained from a mouse immunized with a purified, NSO-induced recombined rat CRP, was obtained from R&D Systems Inc. A glass slide was obtained from Coring Inc. (Kennebunk, ME, USA). A water-soluble biotinylation reagent (sulfosuccinimidyl-6-(biotinamido)hexanoate: sulfo-NHS-LC-biotin) was obtained from Pierce Biotechnology, Inc. (Rockford, IL, USA). 3-aminopropyltrimethoxysilane (APTMS), glutaraldehyde, and bovine serum albumin (BSA) were obtained from Sigma-Aldrich Chemical Co.(St. Louis, MO, USA). Other compounds used in the following experiments were guaranteed by various suppliers, and throughout experiments, double distilled water was used.

### Example 1: Immobilization of antigen on slide treated with APTMS-GA

An antigen was immobilized on a slide as follows. That is, for surface cleaning, a slide was immersed in a piranha solution (H₂SO₄:H₂O₂ = 3:1, volumetric ratio) for 10 minutes, washed with distilled water, sonicated in distilled water for 5 minutes, washed by hybridization with hot water having a temperature of 90°C for 1 hour, placed on kimwipes, and then, dried for 30 minutes at room temperature.

To perform silanization to introduce an amino group (-NH₂), which is needed to immobilize an antigen, to the surface of the slide, the surface-cleaned slide was placed in a petri dish containing an APTMS solution in which 10% APTMS was dissolved in acetone and a reaction was performed at room temperature for 1 hour. Front and rear sides of the slides were sequentially washed with a 50 mM phosphate buffer solution (pH 7.4) and distilled water, three times for each, and then, immersed in a beaker containing distilled water for 1 minute. The resultant slide was taken away therefrom, and then, dried on kimwipes for 30 minutes at room temperature, heat treated in a convection oven at a temperature of 120°C for 5 hours, and left to sit for cooling, and a fluorescent image thereof was obtained (see FIGS. 5A, 5B, and 5C). As shown in FIGS. 5A, 5B, and 5C, it was confirmed that inhibitory spots of a contaminating material, such as impurities, present on the surface of a slide before treatment with a piranha solution were removed by a piranha treatment, and even after silanization with APTMS, a fluorescent image of inhibitory spots was not observed.

To activate the APTMS-modified slide, the slide was immersed in distilled water for 15 minutes, and taken away therefrom and placed in a petri dish containing glutaraldehyde 2.5% solution, and a reaction was performed for 1 hour to enable a protein to bind to the surface of the slide. Front and rear surfaces of the activated slide were washed three times with 50mM phosphate buffer solution (pH 7.4) and then, immersed in a beaker containing distilled water for 1 minute. The slide was taken away therefrom and placed on kimwipes and dried for 30 minutes at room temperature, and a fluorescent image thereof also shows no inhibitory spots on the surface of the GA-activated slide (see FIG. 5D).

To immobilize an antigen (CRP, C-reactive protein) on a slide, first, a C-reactive protein was dissolved at a centration of 0.1 mg/mℓ in a 50 mM phosphate buffer solution (pH 7.4) to prepare an antigen solution for immobilization. A petri dish containing the glutaraldehyde-activated slide was placed on a spotting guide having a lattice structure, and 20*µ*ℓ of the antigen solution was spotted on a portion of the slide corresponding to a spot point on the spotting guide, and then, the petri dish was covered with a cover and a reaction was performed for 1 hour to immobilize an antigen, and then, front and rear surfaces of the slide were washed three times with 50mM phosphate buffer solution (pH 7.4) and immersed in a beaker containing distilled water for 1 minute. To block a portion of the glutaraldehyde-activated slide subjected to the immobilizing of an antigen which did not react with the antigen, the slide was placed in a small plastic petri dish containing 1% bovine serum albumin (BSA) solution in which 1% bovine serum albumin was dissolved in a 50 mM phosphate buffer solution (pH 7.4) and the petri dish was covered with a cover and a reaction was performed for 1 hour. Then, front and rear sides of the slide were washed three times with 50mM phosphate buffer solution (pH 7.4), and the slide was immersed in a beaker containing distilled water for 1 minute, and placed on kimwipes and dried for 30 minutes at room temperature, and a fluorescent image thereof also showed no inhibitory spots on the surface of the BSA-blocking slide after the antigen was immobilized (see FIG. 5E).

### Example 2 (not forming part of the present invention): Immobilization of antigen on slide treated with MTS-GMBS

Immobilizing of an antigen on a slide was performed through the following processes. That is, for surface cleaning, a slide was immersed in a mixed solution including a strong hydrochloric acid (35%) and methanol (70%) at a volumetric ratio of 1:1 for 30 minutes, and then, the slide was washed three times with distilled water. After 30 minutes of immersion in a strong sulfuric acid, front and rear surfaces of the slide were washed three times with distilled water, and washed with boiled distilled water and placed on kimwipes and dried at room temperature for 30 minutes.

To perform silanization to introduce a thiol group (-SH), which is a reaction group needed to immobilize an antigen, to the surface of a slide, 2 mℓ of (3-mercaptopropyl)trimethoxy silane (MPTMS) was added to 98 mℓ of toluene to prepare a 2% MPTMS solution. The slide was placed on a petri dish containing the solution and a reaction was performed at room temperature for 2 hours. Thereafter, the slide was washed three times with toluene, and then, placed on kimwipes and dried for 30 minutes at room temperature.

To activate the slide modified with MPTMS, 28 mg of GMBS was dissolved in 100 *µ*ℓ of N,N-Dimethylformamide (DMF, chromosllvr^{®}Plus, St. Louis, MO, USA, HPLC≥99.9%) and 49.9 mℓ of ethanl was added thereto to prepare a 2 mM GMBS solution. The silanized slide was placed on a petri dish containing the 2 mM GMBS solution and a reaction was performed at room temperature for 1 hour. Front and rear sides of the slide were washed three times with distilled water and a 50 mM phosphate buffer solution (pH 7.4), and dried on kimwipes for 30 minutes at room temperature.

To immobilize an antigen (CRP, C-reactive protein) on the slide, first, a 0.1 mg/mℓ C-reactive protein was dissolved in 50 mM phosphate buffer solution(pH 7.4) to prepare an antigen solution for immobilizing. A petri dish containing the GMBS-activated slide was placed on a spotting guide, and 20 *µ*ℓ of the antigen solution was spotted on a portion of the slide corresponding to a spot point on the spotting guide, and then, the petri dish was covered with a cover and a reaction was performed for 1 hour to immobilize an antigen, and then, front and rear surfaces of the slide were washed three times with 50 mM phosphate buffer solution (pH 7.4) and immersed in a beaker containing distilled water for 1 minute. To block a portion of the GMBS-activated slide subjected to the immobilizing of an antigen which did not react with the antigen, the slide was placed in a small plastic petri dish containing 1% bovine serum albumin (BSA) solution in which 1% bovine serum albumin was dissolved in a 50 mM phosphate buffer solution (pH 7.4), and the petri dish was covered with a cover and a reaction was performed for 1 hour. Then, front and rear sides of the slide were washed three times with 50 mM phosphate buffer solution (pH 7.4), and the slide was immersed in a beaker containing distilled water for 1 minute, and placed on kimwipes and dried for 30 minutes at room temperature.

A fluorescent image of the slide was taken at the respective steps of the process of immobilizing an antigen on a MTS-GMBS treated slide, that is, surface cleaning, silanization, activation, immobilization of an antigen, and BSA blocking. As a result, it was confirmed that inhibitory spots, which inhibit sensor measuring on the surface of the slide, were not shown in all the fluorescent images, excluding that of the slide before the treatment with the mixed solution including a strong hydrochloric acid and methanol (1:1, volumetric ratio).

### Example 3 (not forming part of the present invention): Preparation of FSNP modified with SA

2 mg of FSNP and 10 mℓ of ethanol were added to a cap tube having a capacity of 25 mℓ and the mixture was shaken well, and then, sonicated to completely dissolve FSNP in ethanol for 15 minutes. Thereafter, 100 *µ*ℓ of MPTMS was added to the cap tube and a reaction was performed at room temperature for 4 hours while stirring at a low sped of 100 rpm. The reaction mixture was centrifuged at a temperature of 4°C for 30 minutes at a rate of 13000 rpm and the obtained precipitate was washed three times with ethanol, and after each washing, centrifuging was performed under the same conditions as described above to obtain precipitates. The cap of the tube was separated and the tube was lightly covered with an aluminum foil and dried at room temperature. Each of the precipitates was dissolved in 4 mℓ of 50 mM phosphate buffer solution (pH 7.4) to prepare a thiol-modified FSNP solution.

0.25 mg of SA-msaleimide and 5 mℓ of 50 mM phosphate buffer solution (pH 7.4) were added to a cap tube having a capacity of 25 mℓ and the SA-maleimide was dissolved to prepare a SA-maleimide solution. Then, 1 mℓ of the thiol-modified FSNP solution was added thereto and while continuously stirring at a low speed of 100 rpm, a reaction was performed at room temperature for 2 hours. The reaction product was centrifuged at a temperature of 4°C for 20 minutes at a rate of 10000 rpm to obtain a precipitate. The precipitate was washed three times with a 50 mM phosphate buffer solution (pH 7.4), and after each washing, centrifuging was performed under the same conditions as described above. The precipitate was re-suspended in 2 mℓ of a 50 mM phosphate buffer solution (pH7.4). The result was added to a cap tube, wrapped with an aluminum foil, refrigerated, and reacted with a biotinylated antibody described below.

### Example 4 (not forming part of the present invention): Biotinylation of antibody

1 mg vial of sulfosuccinimidyl-6-(biotinamido)hexanoate (sulfo-NHS-LC-biotin) that is a water-soluble biotinylation reagent, which had been refrigerated, was moved to a room temperature condition, and 30-700 *µ*ℓ of 0.1 M phosphate buffered saline (pH 7.2) was dissolved in 30-700 *µ*ℓ vial of monoclonal anti-rat CRP antibody. 180 *µ*ℓ of distilled water was added to 1 mg vial of sulfo-NHS-LC-biotin to prepare a 10 mM sulfo-NHS-LC-biotin solution, and then, 6.67 *µ*ℓ of the mixture was added to the antibody solution to control a molecular ratio of biotin to an antibody to be 20:1. A vial containing the reaction mixture was softly shaken, and then, placed in an eppendorf tube rack and then in iced water and a reaction was performed for 2 hours. Then, the reaction mixture was loaded into a slide-A-lyzer kit (Pierce Biotechnology, Inc) and then, dialyzed with respect to a 0.1M phosphate buffered saline (pH 7.2) overnight and an unreacted reagent was removed and a dialyzate remaining inside the dialysis membrane was recovered. and then. the total volume of the dialyzate was controlled to be 1 mℓ by using the same phosphate buffered saline to prepare biotinylated antibody.

### Example 5 (not forming part of the present invention): Preparation of antibody to which nanomaterial bonds

A SA-modified FSNP solution was sonicated for 15 minutes and then, 400 *µ*ℓ of the result was added to 2 mℓ of a 0.1 mg/mℓ biotinylated antibody solution, and while stirring at time intervals of 30 minutes at a low speed of 100 rpm, each for 5 minutes, a reaction was performed at room temperature for 2 hours. The reaction product was centrifuged at a temperature of 4°C for 20 minutes at a rate of 10000 rpm, and a supernatant was discarded and a precipitate was collected. The precipitate was washed three times with 50 mM phosphate buffer solution (pH 7.4), and after each washing, centrifuging was performed under the same conditions as described above to obtain a precipitate. The precipitates were re-suspended in 0.8 mℓ of 50 mM phosphate buffer solution (pH 7.4).

### Example 6 (not forming part of the present invention): mixing of specimen solution and label antibody

A C-reactive protein antibody solution to which FSNP was bonded as a nanomaterial, which was prepared based on a specific bonding between biotin and SA, was added to various concentrations of a C-reactive protein sample solution which was prepared by dilution using 50 mM phosphate buffer solution (pH 7.4) contained in an eppendorf tube, wherein amounts of the C-reactive protein antibody solution and the C-reactive protein sample solution were the same, and these solutions were mixed and a reaction was performed room temperature for 1 hour.

### Example 7: Indirect Competitive Reaction

A petri dish containing a slide as a biotransducer in which a C-reactive protein as an antigen was immobilized and adsorption of a non-selective protein was minimized by BSA blocking according to Examples 1 and 2, was placed in a spotting guide having a lattice structure, and mixing was performed where the antigen was immobilized on the slide, and 20 *µ*ℓ of a mixture including a target protein which remained stationary at room temperature for 1 hour and collected from a specimen and the labeled antibody was accurately spotted, and the resultant structure was covered and a reaction was performed at room temperature for 2 hours to perform an indirect competitive reaction between the antigen immobilized on the slide and a target protein collected from a specimen against the FSNP-labeled antibody. After a reaction was stopped, an unreacted antibody and antigen were removed by washing three times front and rear sides of the slide with distilled water, and placing the slide in a beaker containing distilled water for 1 minute.

### Example 8: Fluorescent image assay

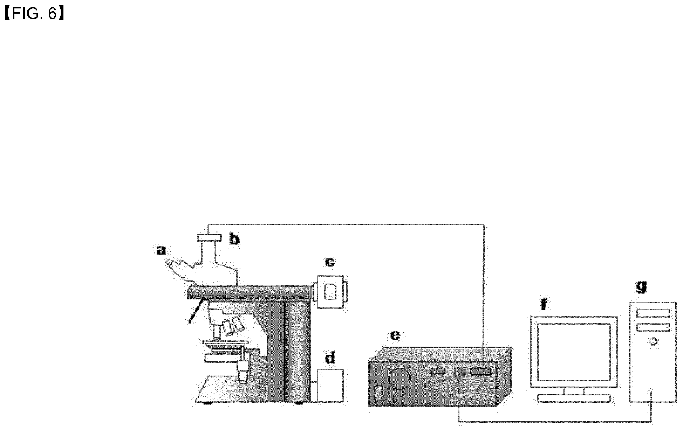

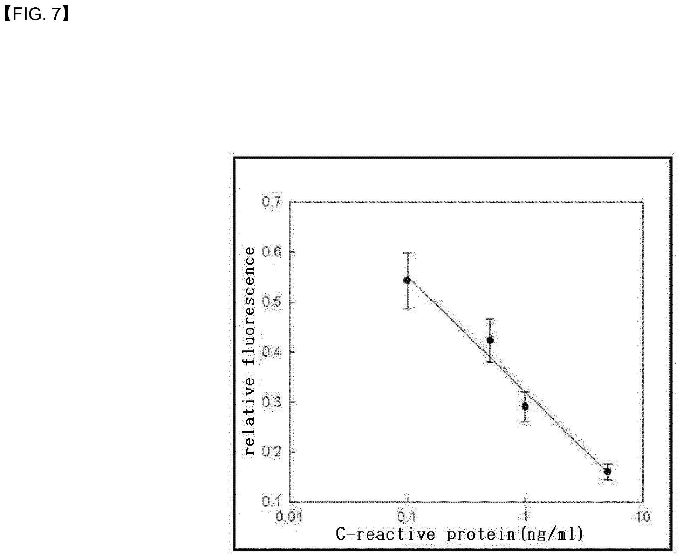

The slide subjected to the indirection competitive reaction according to Example 7 was placed in an incubator and dried. A fluorescent image assay was performed on the dried slide by using a fluorescence microscope, and fluorescent intensity and the number of fluorescent particles were measured. FIG. 6 is a view of an apparatus for fluorescent assay, and a fluorescent image thereof was obtained and showed fluorescent spots of a FSNP-labeled antibody binding to the surface of the slide subjected to an indirect competitive reaction (see FIG. 5F). FIG. 7 shows a graph of a calibration curve obtained by measuring a relative fluorescent degree obtained from a concentration-dependent fluorescent image of a C-reactive protein by using an FSNP-labeled antibody. When the concentration of CRP in a specimen increased, more CRP reacted with a labeled antibody, and thus, the binding between the antigen immobilized on the slide and the labeled antibody decreased. In addition, the graph showed linearity in a very low concentration range of the C-reactive protein. This shows that a detection limit when CRP is measured by using the immuno-fluorescence slide is a very high of 0.1 to 1.0 ng/mℓ.

### Example 9: Preparation of immuno-fluorescence slide

A disposable immuno-fluorescent slide was prepared by surface-modifying a transparent slide which had a size of 30X70 mm and was used for a microscope with 3-aminopropyltrimethoxysilane or 3-mercaptopropyltrimethoxysilane-N-gamma maleimidobutyryloxy succin imide ester and immobilizing 1-100 *µ*ℓ of 0.01-0.5 mg/mℓ C-reactive protein thereon according to Examples 1 to 8.

## Claims

1. A method of preparing a C-reactive-protein-immobilized immuno-fluorescence slide, the method comprising:
- immobilizing a C-reactive protein on a slide to prepare a protein chip; wherein the immobilizing of the C-reactive protein on the slide comprises:
modifying the slide with 3-aminopropyltrimethoxysilane to prepare a modified slide;
hydrating the slide modified with 3-aminopropyltrimethoxysilane;
activating the hydrated modified slide by using a glutaraldehyde solution;
dissolving target C-reactive protein at a concentration of 0.01-0.5 mg/ml in a 30-70 mM phosphate buffer solution, with pH 6.5-7.8 to prepare a C-reactive protein solution for immobilization;
placing a petri dish comprising the glutaraldehyde-activated slide on a spotting guide and spotting 1-100 *µ*ℓ of the C-reactive protein solution on spotting points; and
performing an immobilization reaction on the glutaraldehyde-activated slide prepared as described above for 1-6 hours to immobilize the C-reactive protein.

2. A C-reactive-protein-immobilized immuno-fluorescence slide prepared by the method of claim 1.

3. An in-vitro method of measuring a C-reactive protein in a specimen by using the immuno-fluorescence slide of claim 2.

4. The in-vitro method of claim 3, wherein the method comprises
- labeling an antibody that specifically binds to C-reactive protein, with a fluorescent nanoparticle;
- mixing a target C-reactive protein obtained from a specimen with the fluorescent labeled antibody,
- reacting the mixture with the C-reactive-protein-immobilized immuno-fluorescence slide, and
- assaying C-reactive protein by measuring fluorescent intensity or the number of fluorescent particles by using a fluorescence microscope.

## Patentansprüche

1. Verfahren zum Herstellen eines mit C-reaktivem Protein immobilisierten Immunfluoreszenzträgers, wobei das Verfahren umfasst:
Immobilisieren eines C-reaktiven Proteins auf einem Träger, um einen Proteinchip herzustellen; wobei das Immobilisieren des C-reaktiven Proteins auf dem Träger umfasst:
Modifizieren des Trägers mit 3-Aminopropyltrimethoxysilan zum Herstellen eines modifizierten Trägers;
Hydratisieren des mit 3-Aminopropyltrimethoxysilan modifizierten Trägers;
Aktivieren des hydratisierten modifizierten Trägers unter Verwendung einer Glutaraldehydlösung;
Auflösen von C-reaktivem Zielprotein in einer Konzentration von 0,01-0,5 mg/ml in einer 30-70 mM Phosphatpufferlösung, mit einem pH 6,5-7,8, um eine C-reaktive Protein-Lösung zur Immobilisierung herzustellen;
Stellen einer Petrischale, umfassend den Glutaraldehyd-aktivierten Träger, auf eine Betupfungsvorrichtung und Betupfen von 1-100 µl der C-reaktiven Proteinlösung auf Betupfungspunkte; und
Durchführen einer Immobilisierungsreaktion auf dem Glutaraldehyd-aktivierten Träger, hergestellt wie oben beschrieben, für 1-6 Stunden zum Immobilisieren des C-reaktiven Proteins.

2. Mit C-reaktivem Protein-immobilisierter Immunfluoreszenzträger, hergestellt durch das Verfahren nach Anspruch 1.

3. In-vitro-Verfahren zum Messen eines C-reaktiven Proteins in einer Probe unter Verwendung des Immunfluoreszenzträgers nach Anspruch 2.

4. In-vitro-Verfahren nach Anspruch 3, wobei das Verfahren umfasst:
- Markieren eines Antikörpers, der spezifisch an das C-reaktive Protein bindet, mit einem Fluoreszenznanopartikel;
- Mischen eines C-reaktiven Zielproteins, erhalten aus einer Probe, mit dem fluoreszierend markierten Antikörper,
- Reagieren der Mischung mit dem mit C-reaktivem Protein-immobilisierten Immunfluoreszenzträger, und
- Testen auf C-reaktives Protein unter Messen von Fluoreszenzintensität oder der Anzahl an fluoreszierenden Partikeln unter Verwendung eines Fluoreszenzmilcroslcops.

## Revendications

1. Procédé de préparation d'une lame d'immunofluorescence à protéine C réactive immobilisée, le procédé comprenant :
- l'immobilisation d'une protéine C réactive sur une lame pour préparer une puce de protéines ; dans lequel l'immobilisation de la protéine C réactive sur la lame comprend :
la modification de la lame avec du 3-aminopropyltriméthoxysilane pour préparer une lame modifiée ;
l'hydratation de la lame modifiée avec du 3-aminopropyltriméthoxysilane ;
l'activation de la lame modifiée et hydratée en utilisant une solution de glutaraldéhyde ;
la dissolution de la protéine C réactive cible à une concentration de 0,01 à 0,5 mg/mL dans une solution tampon de phosphate de 30 à 70 mM, avec un pH de 6,5 à 7,8 pour préparer une solution de protéine C réactive pour immobilisation ;
le placement d'une boîte de pétri comprenant la lame activée par glutaraldéhyde sur un guide d'application et l'application de 1 à 100 µL de la solution de protéine C réactive sur des points d'application ; et
la réalisation d'une réaction d'immobilisation sur la lame activée par glutaraldéhyde préparée comme décrit ci-dessus pendant 1 à 6 heures pour immobiliser la protéine C réactive.

2. Lame d'immunofluorescence à protéine C réactive immobilisée préparée par le procédé de la revendication 1.

3. Procédé in vitro de mesure d'une protéine C réactive dans un spécimen en utilisant la lame d'immunofluorescence de la revendication 2.

4. Procédé in vitro selon la revendication 3, dans lequel le procédé comprend
- le marquage d'un anticorps qui se lie spécifiquement à la protéine C réactive, avec une nanoparticule fluorescente ;
- le mélange d'une protéine C réactive cible obtenue à partir d'un spécimen avec l'anticorps marqué fluorescent,
- la réaction du mélange avec la lame d'immunofluorescence à protéine C réactive immobilisée, et
- l'analyse de la protéine C réactive en mesurant l'intensité fluorescente ou le nombre de particules fluorescentes en utilisant un microscope à fluorescence.
